# EUROPEAN PATENT APPLICATION

(11) **EP 4 852 011 A1**
(43) Date of publication of application: **09.12.2026**
(21) Application number: 26165664.9
(22) Date of filing: 18.03.2026
(51) Int. Cl.: G16H 30/20, G16H 30/40, G16H 50/20, G16H 15/00, G16H 50/70

(54) **MEDICAL IMAGE ANNOTATING METHOD AND SYSTEM**

(30) Priority: 04.06.2025 TW 114120785
(71) Applicant: Acer Incorporated, New Taipei City 221 (TW); Acer Medical Inc., New Taipei City 22181 (TW)
(72) Inventor: CHUNG, Shang-Lin, New Taipei City 221 (TW); LI, Chien-Hung, New Taipei City 22181 (TW); CHENG, Meng-Che, New Taipei City 22181 (TW)
(74) Representative: Haseltine Lake Kempner LLP

(57) **Abstract**

Provided are a medical image annotating method and a system (100). The medical image annotating method includes the following steps. According to disease process information (DI) and user indication (I1), a first pattern of a medical image (IMG0) is masked to generate a first image (IMG1). According to the disease process information (DI), the first image (IMG1) is applied to multiple color models to generate multiple second images (IMG2), such that a user (UR) interprets symptom pattern information in the second images (IMG2) according to the disease process information (DI) to provide interpretation indication (I2). According to the interpretation indication (I2) and preset annotation information, the medical image (IMG0) is annotated to generate an annotation result (R1), thereby consistency and accuracy of the annotation result (R1) is increased.

## Description

### BACKGROUND

### Technical Field

The disclosure relates to an image processing method, and particularly relates to a medical image annotation method and a system.

### Related Art

**In** medical applications, through doctors annotating medical images, artificial intelligent (AI) systems may use the annotation results as a basis for training and validating AI models. When different doctors do not reach consensus on the interpretation of diseases, the annotation results of the diseases have high inconsistency, thereby affecting the stability and accuracy of AI model recognition.

Taking glaucoma as an example, doctors may give different interpretation results for the same fundus image due to differences in clinical experience. In addition, based on factors such as the disease process of glaucoma and other comorbid diseases (for example, cataracts), it is difficult for doctors to interpret symptoms such as retinal nerve fiber layer (RNFL) and optic cup/disc based on fundus images, thereby increasing the probability of misdiagnosis. Thus, for medical images, how to improve the consistency and accuracy of annotation results is one of the issues to be resolved.

### SUMMARY

Embodiments of the disclosure provide a medical image annotation method that may improve the consistency and accuracy of annotation results.

The medical image annotation method of embodiments of the disclosure includes the following steps. According to disease process information and user indication, a first pattern of a medical image is masked to generate a first image. According to the disease process information, the first image is applied to multiple color models to generate multiple second images, such that a user interprets symptom pattern information in the second images according to the disease process information to provide interpretation indication. According to the interpretation indication and preset annotation information, the medical image is annotated to generate an annotation result.

Embodiments of the disclosure further provide a medical image annotating system. The medical image annotating system includes a storage device and a processor. The storage device is configured to store medical images. The processor is coupled to the storage device. The processor is configured to execute the medical image annotation method.

Based on the above, the medical image annotation method and the medical image annotating system of embodiments of the disclosure may assist doctors in interpreting masked medical images (that is, first images) by masking portions of medical images based on disease process information. By applying different color models to the first image (that is, multiple second images), the medical image annotating system may further enhance patterns corresponding to symptom structures in the multiple second images. Thus, the medical image annotating system may obtain annotation results with high consistency and accuracy.

To make the foregoing features and advantages of the disclosure more comprehensible, embodiments are specifically provided below and described in detail with accompanying drawings as follows.

### BRIEF DESCRIPTION OF THE DRAWINGS

FIG. 1 is a circuit block diagram of a medical image annotating system according to an embodiment of the disclosure.
FIG. 2 is a flowchart of a medical image annotation method according to an embodiment of the disclosure.
FIG. 3A and FIG. 3B are flowcharts of the medical image annotation method according to another embodiment of the disclosure.
FIG. 4 is a masked medical image according to the embodiments of FIG. 3A and FIG. 3B of the disclosure.
FIG. 5A to FIG. 5B are medical images drawn according to the embodiments of FIG. 3A and FIG. 3B of the disclosure.
FIG. 6A to FIG. 6D are medical images drawn according to the embodiments of FIG. 3A and FIG. 3B of the disclosure.
FIG. 7A to FIG. 7B show medical images applied with multiple color models according to the embodiments of FIG. 3A and FIG. 3B of the disclosure.
FIG. 8A to FIG. 8B are annotation results illustrated according to the embodiments of FIG. 3A and FIG. 3B of the disclosure.
FIG. 9 is a flowchart of the medical image annotation method illustrated according to another embodiment of the disclosure.
FIG. 10 is a flowchart of the medical image annotation method illustrated according to another embodiment of the disclosure.

### DESCRIPTION OF THE EMBODIMENTS

Some embodiments of the disclosure will be described in detail with reference to the accompanying drawings. The reference numerals referenced in the following description, when the same reference numerals appear in different drawings, will be regarded as the same or similar elements. These embodiments are merely a part of the disclosure and do not disclose all possible implementations of the disclosure. More specifically, these embodiments are merely examples within the scope of the appended claims of the disclosure.

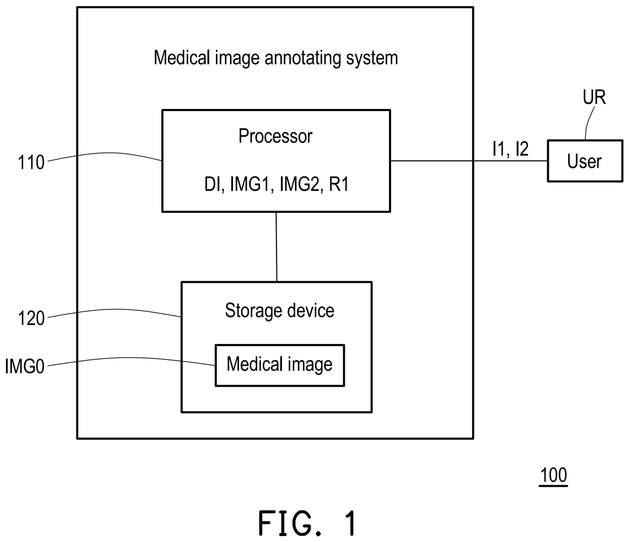

FIG. 1 is a circuit block diagram of a medical image annotating system according to an embodiment of the disclosure. Referring to FIG. 1, a medical image annotating system 100 may be applied to various diseases. The medical image annotating system 100 is capable of interactively annotating a medical image IMG0 with a user UR, and is capable of ensuring consistency and accuracy of an annotation result R1 among different users UR. As such, other application systems may use the annotation result R1 as a training set and/or validation set, and train models therein according to the annotation result R1 to implement identification applications of symptom structures.

In the present embodiment, one or more users (for example, doctors) UR may operate electronic devices (not shown) to invoke the medical image annotating system 100 through an application programming interface (API) for interpretation and annotation. The electronic devices may be, for example, mobile phones, tablet computers, laptop computers, or desktop computers.

In the present embodiment, the medical image annotating system 100 includes a processor 110 and a storage device 120. The processor 110 is coupled to the storage device 120. The storage device 120 stores the medical image IMG0. The storage device 120 may be, for example, dynamic random access memory (DRAM), flash memory, non-volatile random access memory (NVRAM), or a combination of these memories.

In the present embodiment, the processor 110 is coupled to the electronic device operated by the user UR to interactively access various information (including user indication I1, interpretation indication I2, and annotation result R1) with the user UR. The processor 110 is further coupled to a database (not shown) to access the medical image IMG0. The database may be, for example, a database disclosed by an institutional review board (IRB) or a public dataset.

The processor 110 may be, for example, a signal converter, a field programmable gate array (FPGA), a central processing unit (CPU), or other programmable general-purpose or special-purpose microprocessors, a digital signal processor (DSP), a programmable controller, an application specific integrated circuit (ASIC), a programmable logic device (PLD), or other similar devices or combinations of these devices, which may load and execute computer program-related firmware or software to implement annotation and various calculation functions.

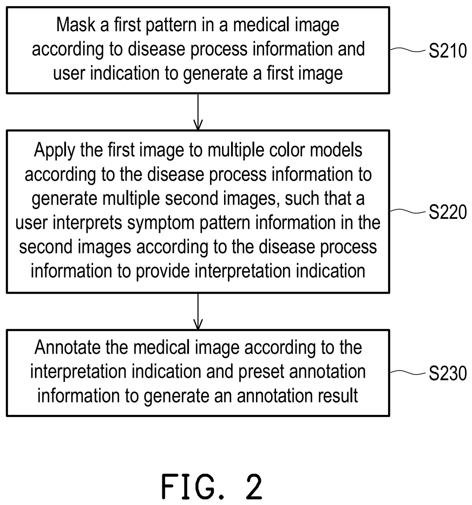

FIG. 2 is a flowchart of a medical image annotating method according to an embodiment of the disclosure. Referring to FIG. 1 and FIG. 2, the medical image annotating system 100 executes Steps S210 to S230 through the processor 110 to implement the medical image annotating method. The order of these Steps S210 to S230 is merely for illustrative purposes.

In Step S210, the processor 110 masks a first pattern in the medical image IMG0 according to the disease process information DI and the user indication I1 provided by the user UR to generate a first image IMG1. That is, the first image IMG1 is the medical image IMG0 having a masked pattern (for example, the first pattern presented in all black or all white).

In the present embodiment, the disease process information DI indicates the interpretation target of the disease at a specific disease process. Based on the disease process information DI, the user indication I1 may be, for example, indication information for the user UR to mask the medical image IMGO, so as to indicate how to mask a portion of the medical image IMG0 to obtain the first image IMG1. The masked first pattern indicates the structure where the disease may develop symptoms outside the specific disease process.

Taking glaucoma as an example, the disease process information DI may be, for example, using early symptoms of glaucoma as the interpretation target. In the medical image IMGO, the masked first pattern may be, for example, a portion of the image corresponding to the optic cup or optic disc, and the unmasked other patterns may be, for example, a portion of the image including the retinal nerve fiber layer (RNFL).

In Step S220, the processor 110 applies the first image IMG1 to multiple color models according to the disease process information DI to generate multiple second images IMG2. The multiple color models are configured to perform different image processing on the first image IMG1 and represent the first image IMG1 in various color modes. The multiple color models include an original color image model, a single color channel image model, and a contrast-enhanced image model.

In this way, the user UR interprets symptom pattern information in the multiple second images IMG2 according to the disease process information DI to provide interpretation indication I2. That is, based on the interpretation target, the user UR interprets whether the medical image IMG0 having the masked pattern (that is, the first pattern) and represented in the various color modes has a symptom structure. The result of the interpretation may be, for example, represented by the interpretation indication I2, so as to indicate a second pattern corresponding to the symptom structure in the medical image IMG0.

In Step S230, the processor 110 annotates the medical image IMG0 according to the interpretation indication I2 provided by the user UR and preset annotation information to generate the annotation result R1. The preset annotation information may be, for example, conventional settings capable of identifying physiological structures. That is, based on the interpretation indication I2, the processor 110 effectively annotates the medical image IMG0 to obtain the annotation result R1.

It is worth mentioning that by masking a portion of the medical image IMG0 through the processor 110, the medical image annotating system 100 may assist the user UR in interpreting the symptom structure associated with the disease process in the first image IMG1 according to the disease process information DI. By applying the first image IMG1 to different color models through the processor 110, the medical image annotating system 100 may enhance the pattern corresponding to the symptom structure in the multiple second images IMG2 to improve the consistency and accuracy of the interpretation of the user UR within a reasonable working time. In this way, the medical image annotating system 100 may provide the user UR with flexible guidance strategies according to the first image IMG1 and the multiple second images IMG2, and simultaneously perform basic interpretation processes on the medical image IMG0 according to various indications I1 to I2 to improve the consistency and accuracy of the annotation result R1.

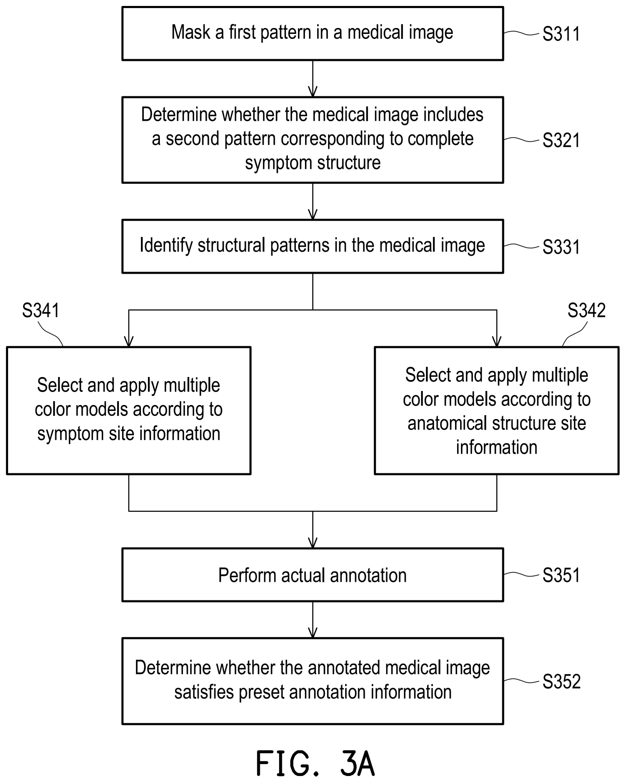

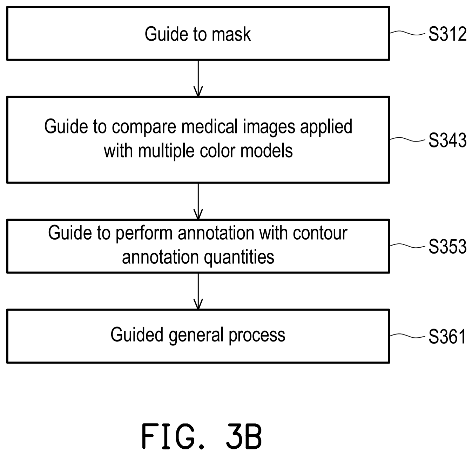

FIG. 3A and FIG. 3B are flowcharts of the medical image annotating method according to another embodiment of the disclosure. Referring to FIG. 3A and FIG. 3B, the medical image annotating system (for example, the medical image annotating system 100 shown in FIG. 1) executes Steps S311 to S361 through a processor to implement the medical image annotating method.

Since medical images may include structural patterns having a not-entirely-dependent relationship with the disease process (for example, the first pattern in the medical image IMG0 shown in FIG. 1), it is difficult for the user to accurately interpret symptom information. That is, the medical images include structural patterns corresponding to early-stage symptoms, and simultaneously include another structural pattern corresponding to late-stage symptoms, and/or other structural patterns corresponding to comorbidities or other factors.

Taking glaucoma as an example, early symptoms include the beginning of retinal nerve fiber layer defects. As the disease process progresses, the cup-to-disc ratio (VCDR) in the vertical direction gradually increases as a late-stage symptom. Assuming a patient has glaucoma combined with high myopia and cataracts, the symptoms of retinal nerve fiber layer defects may be diminished. When the increase in the VCDR is not apparent, it is difficult for doctors to interpret the symptoms associated with glaucoma in the fundus image of the patient, resulting in delayed medical treatment for the patient.

In order to reduce the difficulty of interpreting medical images, taking early symptoms as the interpretation target as an example, the medical image annotating system may mask structural patterns corresponding to late-stage symptoms in medical images. In this way, doctors may focus on interpreting medical images having early symptoms. In addition, the medical image annotating system may ensure that different doctors are not affected by late-stage symptoms when interpreting early symptoms, thereby improving consistency of interpretation process and interpretation results among different doctors.

Specifically, in Step S311, the processor masks the first pattern in the medical image according to the disease process information (for example, the disease process information DI shown in FIG. 1). The first pattern has a not-entirely-dependent relationship with the symptom structure in the disease process information. That is, the processor masks a pattern of a portion in the medical image that is not entirely dependent on the disease process, so as to ignore patterns that do not belong to the disease process (for example, early symptoms).

In Step S312, the processor provides the medical image having the masked pattern (for example, the first pattern presented in full black or full white) to the user through the electronic device, and guides the user to perform masking on the medical image to obtain user indication (for example, the user indication I1 shown in FIG. 1). That is, after confirming the disease process (for example, early symptoms), the processor finds a pattern (for example, the first pattern) of a portion in the medical image that is not entirely dependent, and guides the user to confirm and/or modify the position and contour of the first pattern, so as to implement the masking function through user feedback. In this way, the processor generates the first image (for example, the first image IMG1 shown in FIG. 1) according to the medical image and the user indication.

In addition, the processor may further analyze public data related to the disease process, and confirm again the not-entirely-dependent relationship between the first pattern and the symptom structure in the disease process information according to the analysis results. Thus, the medical image annotating system may improve the consistency of the annotation process and annotation results.

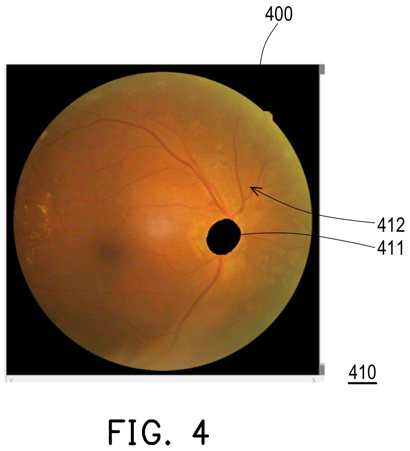

Referring to FIG. 4 together, FIG. 4 is a masked medical image drawn according to the embodiments of FIG. 3A and FIG. 3B of the disclosure. Taking glaucoma as an example, a medical image 400 may be, for example, a fundus image. Based on the disease process information indicating early symptoms, the processor masks a first pattern 411 corresponding to the optic disc in the medical image 400 to generate the first image 410.

Thus, the user may interpret whether the first image 410 has early symptoms of glaucoma, for example, including defects in the retinal nerve fiber layer corresponding to the second pattern 412. When the user completes the interpretation and subsequent annotation of the retinal nerve fiber layer, the processor may turn off the masking function to restore the masked first pattern 411, thereby obtaining the medical image 400 or the annotated medical image 400. Based on the medical image 400, the user may further interpret and annotate the first pattern 411 to continue completing the annotation of the optic disc.

In order to avoid interpreting and annotating defective medical images, the medical image annotating system may first ensure that the portions to be interpreted in the medical image are presented completely and intact. The defective medical image may be, for example, caused by incorrect shooting settings, and has incomplete structural patterns. Thus, the medical image annotating system may avoid misjudgment situations, and simultaneously improve the consistency of interpretation process and interpretation results among different doctors.

In detail, in Step S321, the processor determines whether the medical image includes the second pattern corresponding to the complete symptom structure according to the disease process information, so as to ensure that the pattern of the required interpretation portion in the medical image are complete and intact.

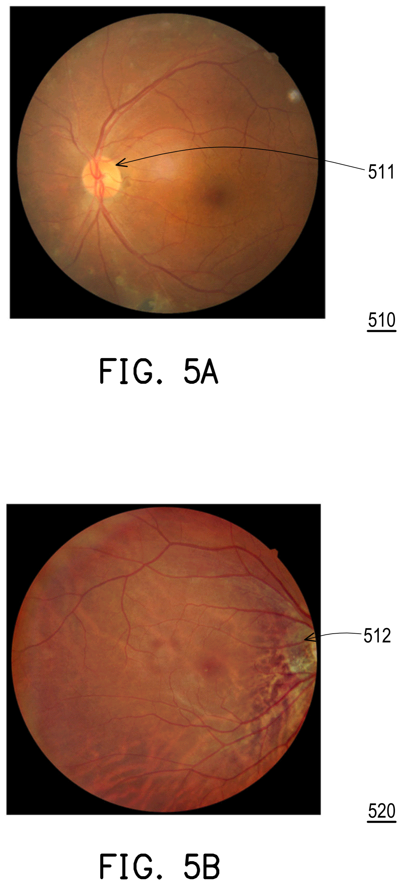

Referring to FIG. 5A to FIG. 5B together, FIG. 5A to FIG. 5B are medical images drawn according to the embodiments of FIG. 3A and FIG. 3B of the disclosure. Taking glaucoma as an example, when the eye gaze position of the patient is fixed on a normal fixation point, a medical image 510 may be, for example, the fundus image shown in FIG. 5A. A second pattern 511 is a closed region corresponding to the complete optic disc structure in the fundus image. The closed region is a complete elliptical region to indicate the optic disc.

On the other hand, when the eye gaze position of the patient is not fixed on a normal fixation point, a medical image 520 may be, for example, the fundus image shown in FIG. 5B. A second pattern 512 is an open region corresponding to the incomplete optic disc structure in the fundus image. Since the edge of the optic disc is not completely captured, the cup-to-disc ratio (VCDR) in the vertical direction may be interpreted, but the rim-to-disc ratio (RDR) at the upper and lower sides cannot be interpreted.

Thus, the processor determines that the medical image 510 includes the second pattern 511 corresponding to the complete symptom structure (that is, the optic disc structure) according to the disease process information (for example, early symptoms of glaucoma), and determines that the medical image 520 includes the second pattern 512 corresponding to the incomplete symptom structure. The processor retains the medical image 510 and discards the medical image 520 according to the determination result. Therefore, the medical image annotating system may avoid doctors interpreting and annotating the medical image 520 having defective symptom structural patterns.

In order to avoid interpreting and annotating blurred medical images, the medical image annotating system may first ensure that the portions to be interpreted in the medical image are presented clearly and visibly. The blurred medical image may be, for example, caused by incorrect or poor shooting conditions, and has unclear structural patterns. Thus, the medical image annotating system may improve the quality of the medical images to be annotated.

In detail, in Step S331, the processor determines whether the medical image includes one or more identifiable structural patterns according to the disease process information. The identifiable structural pattern may be, for example, having clear contours to indicate physiological structures. Thus, the processor may ensure that the pattern of the required interpretation portion in the medical image are clearly visible.

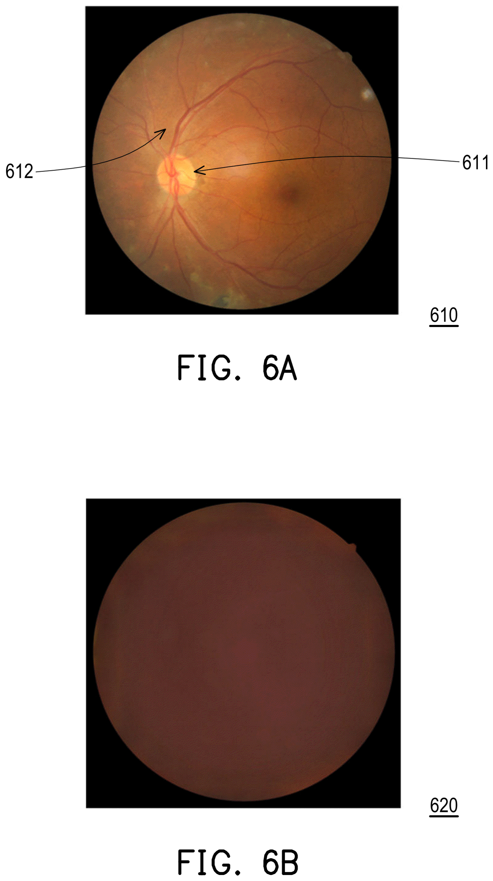

Referring to FIG. 6A to FIG. 6D together, FIG. 6A to FIG. 6D are medical images drawn according to the embodiments of FIG. 3A and FIG. 3B of the disclosure. Taking glaucoma as an example, when a medical image 610 includes identifiable structural patterns, the medical image 610 may be, for example, the fundus image shown in FIG. 6A. The identifiable structural patterns include at least one of an optic disc pattern 611 and a vascular branch pattern 612.

The processor may, for example, identify the optic disc and optic cup contours in the medical image 610. The processor determines that the medical image 610 includes identifiable structural patterns (including the optic disc pattern 611) according to the identification result. Thus, the processor may ensure that the structural pattern corresponding to the optic disc in the medical image 610 is clearly visible.

The processor may further, for example, identify the Nth blood vessel in the medical image 610. The processor determines that the medical image 610 includes identifiable structural patterns (including the vascular branch pattern 612) according to the identification result. Thus, the processor may ensure that the structural pattern corresponding to the nerve fiber layer in the medical image 610 is clearly visible.

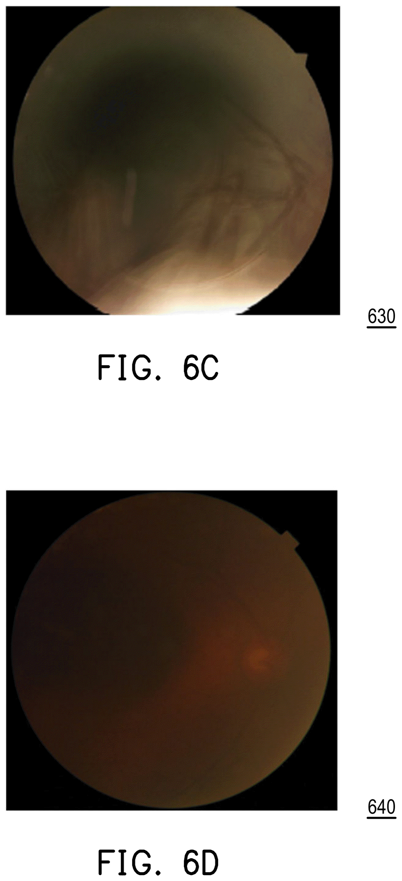

On the other hand, when dirt adheres to the shooting lens, the medical image 620 may be, for example, the fundus image shown in FIG. 6B. When the eyelashes of the patient block the lens, resulting in insufficient light, a medical image 630 may be, for example, the fundus image shown in FIG. 6C. When the lens is out of focus, a medical image 640 may be, for example, the fundus image shown in FIG. 6D. Under these conditions, the processor cannot identify any structure in the medical images 620 to 640.

Thus, the processor determines that the medical image 610 includes identifiable structural patterns 611 to 612 and determines that the medical images 620 to 640 do not include any identifiable structural patterns according to the disease process information (for example, early symptoms of glaucoma). The processor retains the medical image 610 and discards the medical images 620 to 640 according to the determination result. Therefore, the medical image annotating system may avoid doctors from interpreting and annotating the medical images 620 to 640 having blurred symptom structural patterns.

In order to effectively perform interpretation and annotation within normal working time, the medical image annotating system may apply the medical image to different color models to highlight the symptom structures presented in the medical image under various color modes. Thus, the medical image annotating system may provide doctors with the same interpretation process, and simultaneously avoid doctors from ignoring the color model on their own, thereby improving the consistency of interpretation process and interpretation results among different doctors.

It should be noted that the examination methods of medical images include examination for disease-related symptom sites and examination for disease-related anatomical structure sites. These examinations present more image information through changes of the medical image between different color modes compared to in a single color mode. Even for the same medical image, depending on the different sites being examined, the image may be applicable to different color modes and corresponding comparison processes. In order to improve the interpretation consistency of different doctors for the image, the medical image annotating system may be implemented based on Steps S341 to S342 and S343.

For examination of symptom sites, the examination result includes classification information of the sites. The classification information indicates whether the symptoms of the sites show abnormalities and is used to compare with feature patterns of irregular shapes. In Step S341, the processor selects an original color image model and a contrast-enhanced image model as multiple color models according to the symptom site information in the disease process information. The processor applies the medical image having a masked pattern (for example, the first image IMG1 shown in FIG. 1) to the original color image model and the contrast-enhanced image model to generate multiple second images (for example, the second image IMG2 shown in FIG. 1). The multiple second images have highlighted potential one or more irregular symptom site patterns.

On the other hand, for examination of anatomical structure sites, the examination result includes image segmentation information of the sites. The image segmentation information indicates the contour appearance of the sites and is used to compare with feature patterns of specific shapes. In Step S342, the processor selects an original color image model and a single color channel image model as multiple color models according to anatomical structure site information in the disease process information. The processor applies the medical image having the masked pattern (for example, the first image IMG1 shown in FIG. 1) to the original color image model and the single color channel image model to generate multiple second images (for example, the second image IMG2 shown in FIG. 1). The multiple second images have highlighted one or more regular contour site patterns.

In Step S343, the processor provides the multiple second images generated in Steps S341 to S342 to the user through the electronic device, and guides the user to compare the medical images applied with multiple color models (that is, the multiple second images) to interpret whether the multiple second images have symptom structures to obtain the interpretation indication (for example, the interpretation indication I2 shown in FIG. 1). That is, after confirming the disease process (for example, early symptoms), the processor provides medical images applied with different color filters to the user with minimum requirements, and guides the user to confirm and/or modify the color models applied to the medical images, so as to complete interpretation and annotation within acceptable working time through user feedback. Thus, the processor generates annotated medical images according to the medical images and the interpretation indication.

It should be noted that through the guidance strategy provided in Step S343, the processor may select the color models that most need to be compared in specific symptoms according to professional advice fed back by doctors. Thus, the guidance strategy may force all doctors performing annotation to complete comparison of the most basic color models.

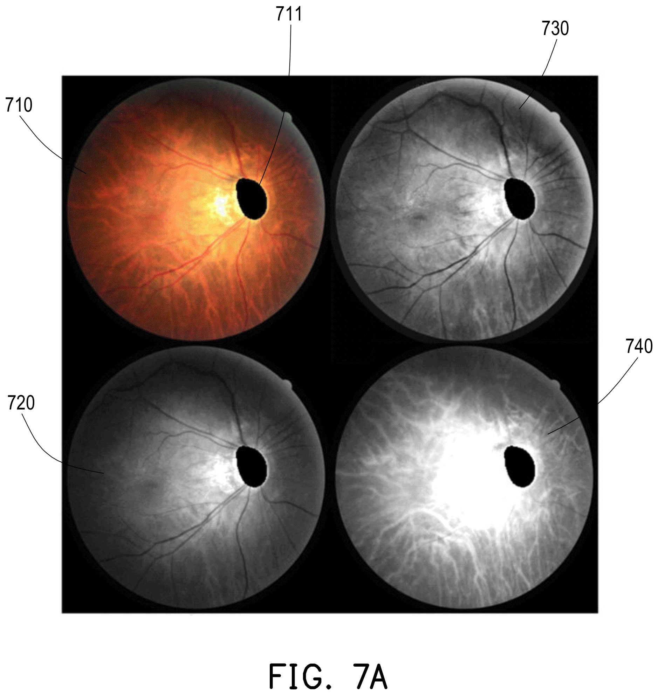

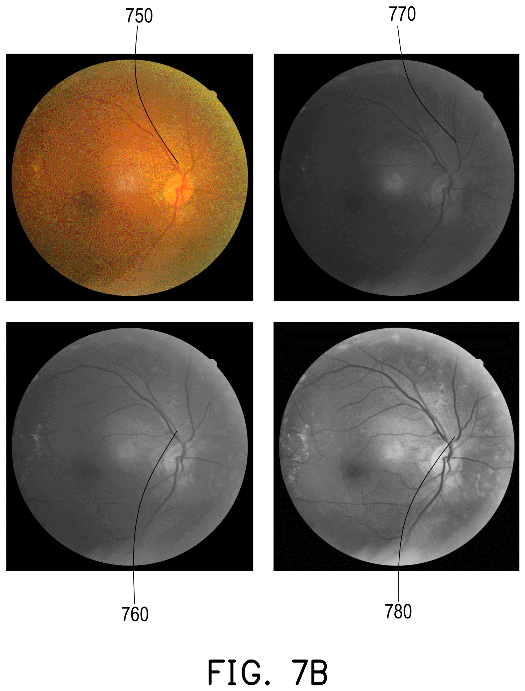

Referring to FIG. 7A and FIG. 7B together, FIG. 7A to FIG. 7B show medical images applied with multiple color models according to the embodiments of FIG. 3A and FIG. 3B of the disclosure, in which the medical images may be, for example, medical images having the masked first pattern, or original medical images.

Taking glaucoma as an example, the processor applies the medical image having a first pattern 711 to an original color image model, a single color channel image model, a contrast enhancement image, and a mixed color channel image model to respectively generate multiple second images 710 to 740. Alternatively, the processor applies the unmasked and magnified medical image to an original color image model, a single color channel image model, a contrast enhancement image, and a mixed color channel image model to respectively generate multiple second images 750 to 780.

For interpretation of the nerve fiber layer, the processor may, for example, apply the medical image having the masked pattern 711 to an original color image model and a contrast-enhanced image model to generate multiple second images 710 and 730. Thus, the user may compare these images 710 and 730, and interpret whether the nerve fiber layer presented in irregular shapes has abnormalities.

For interpretation of the optic disc, the processor may, for example, apply the unmasked and magnified medical image to an original color image model and a single color channel image model to generate multiple second images 750 and 760. Thus, the user may compare these images 750 and 760, and interpret whether the optic disc presented in a specific shape (for example, elliptical) has abnormalities.

To improve annotation efficiency, the medical image annotating system may ensure that the number of annotation points is within a normal range, while simultaneously avoiding contour abnormalities caused by too few annotation points and time-consuming issues caused by too many annotation points. Thus, the medical image annotating system may effectively complete annotation within normal working time, while simultaneously improving consistency of annotation results among different doctors.

In detail, in Step S351, the user performs actual annotation on the multiple second images in Step S341 to S342 through the processor.

In Step S352, according to the interpretation indication in Step S341 to S343, the processor determines whether the annotated medical image satisfies preset annotation information so as to use the annotated medical image as an annotation result (for example, the annotation result R1 shown in FIG. 1). For examination of symptom sites, the processor determines that the annotated medical image of the user conforms to expected interpretation results of the disease. For examination of anatomical structure sites, the processor may convert the annotated medical image of the user into numerical values to indicate whether structural interpretation is abnormal. The numerical values may be represented by contour annotation quantities.

In Step S353, the processor provides the annotated medical image in Step S352 through the electronic device, and guides the user to perform annotation with contour annotation quantities that satisfy preset annotation information (including preset annotation quantities) to obtain interpretation indication (for example, the interpretation indication I2 shown in FIG. 1). The processor annotates the second pattern in the medical image with contour annotation quantities (for example, having minimum annotation point quantities) according to the interpretation indication. The second pattern corresponds to a complete symptom structure. That is, after confirming the disease process (for example, early symptoms), the processor prompts the user with reasonable annotation point quantities with minimum requirements, and guides the user to confirm and/or modify the annotated medical image, thereby completing annotation of symptom structure contours within acceptable working time through user feedback.

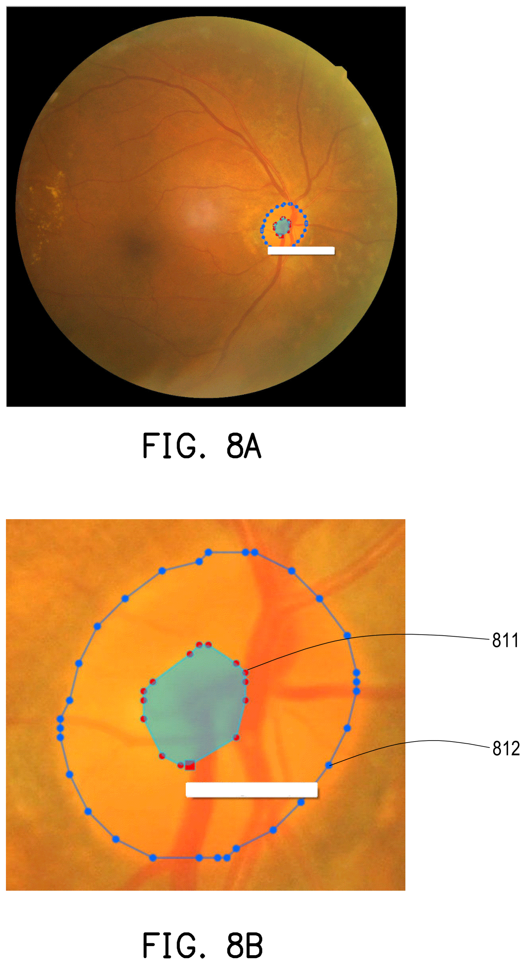

Referring to FIG. 8A to FIG. 8B together, FIG. 8A to FIG. 8B are annotation results illustrated according to the embodiments of FIG. 3A and FIG. 3B of the disclosure, in which FIG. 8B is a partial enlarged view of FIG. 8A. Taking glaucoma as an example, an annotation result 800 includes an annotated symptom pattern 811 and other structural pattern 812. Each pattern 811 to 812 has multiple annotation points that satisfy preset annotation information, and forms identifiable contour structural patterns through the multiple annotation points.

When the medical image annotating system completes annotation of multiple medical images, the medical image annotating system adjusts parameters of multiple Step S311 to S353 to update the annotation method.

In detail, in Step S361, the processor executes Step S311 to S353 based on multiple medical images to generate multiple annotation results according to multiple interpretation indications corresponding to the multiple medical images and preset annotation information. The processor generates multiple guided interpretation options according to comparison results among the multiple annotation results. Thus, the processor updates the multiple guided interpretation options to corresponding Step S312, Step S343, and Step S353 as guided general processes. Different users may perform interpretation according to disease process information based on the multiple guided interpretation options in Step S312, Step S343, and Step S353.

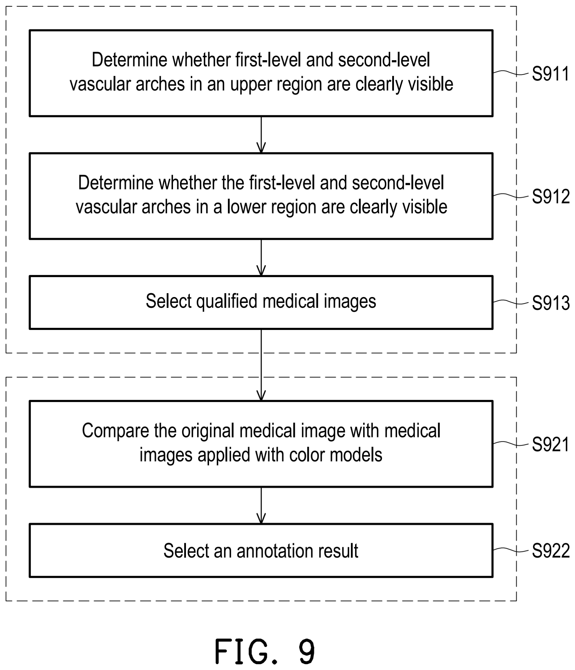

FIG. 9 is a flowchart of the medical image annotation method illustrated according to another embodiment of the disclosure. Referring to FIG. 9, the medical image annotating system (for example, the medical image annotating system 100 shown in FIG. 1) executes Step S911 to S922 through the processor to exemplify how to perform annotation for early symptoms of glaucoma. The medical image to be annotated may be, for example, a fundus image.

To ensure the quality of medical images, the processor executes Step S911 to S913. In detail, in Step S911, the processor determines whether first-level and second-level vascular arches in the upper region of the fundus image are clearly visible. In Step S912, the processor further determines whether first-level and second-level vascular arches in the lower region of the fundus image are clearly visible. In Step S913, based on the determination results in Step S911 to S912, the processor selects fundus images where all determinations are affirmative as qualified medical images.

To effectively interpret symptom structures in fundus images, the processor executes Step S921 to S922. In detail, in Step S921, the processor compares the original medical image with medical images applied with different color models (including original color image model and contrast-enhanced image model). The processor obtains user interpretation indications for the medical images. In Step S922, the processor selects medical images that satisfy preset annotation information in the interpretation indications as annotation results, thereby effectively annotating the retinal nerve fiber layer in fundus images.

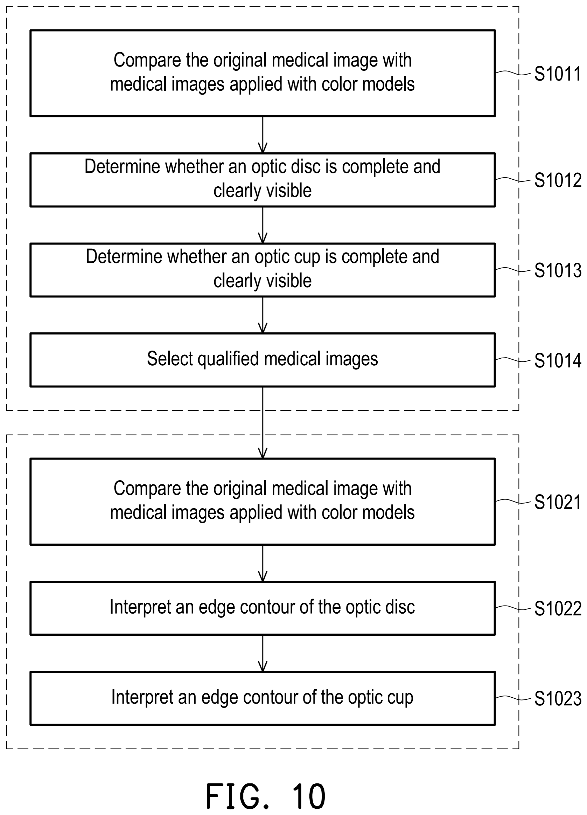

FIG. 10 is a flowchart of the medical image annotation method illustrated according to another embodiment of the disclosure. Referring to FIG. 9, the medical image annotating system (for example, the medical image annotating system 100 shown in FIG. 1) executes Step S1011 to S1023 through the processor to exemplify how to perform annotation for late-stage symptoms of glaucoma. The medical image to be annotated may be, for example, a fundus image.

To ensure the quality of medical images, the processor executes Step S1011 to S1013. In detail, in Step S1011, the processor compares the original medical image with medical images applied with different color models (including original color image model and single color channel image). In Step S1012, the processor determines whether the optic disc is complete and clearly visible in the multiple medical images processed through various filters in Step S1011. In Step S1013, the processor further determines whether the optic cup is complete and clearly visible in the multiple medical images. In Step S1014, based on the determination results in Step S1012 to S1013, the processor selects fundus images where all determinations are affirmative as qualified medical images.

To effectively interpret symptom structures in fundus images, the processor executes Step S1021 to S1023. In detail, in Step S1021, the processor compares the original medical image with medical images applied with different color models (including original color image model and single color channel image). The processor obtains user interpretation indications for the medical images. In Step S1022, the user interprets the edge contour of the optic disc in the image from Step 1021 to provide interpretation indications to the processor. The processor completes annotation of the optic disc according to the interpretation indications and the determination result of whether the edge contour satisfies preset annotation information. In Step S1023, the user interprets the edge contour of the optic cup in the image from Step 1021 to provide interpretation indications to the processor. The processor completes annotation of the optic cup according to the interpretation indications and the determination result of whether the edge contour satisfies preset annotation information.

In summary, the medical image annotation method and the medical image annotating system of the embodiments of the disclosure may introduce guidance strategies and accordingly ensure obtaining annotation results with high consistency. Through providing a unified interpretation process, different users may interpret and annotate medical images based on the medical image annotation method, and reduce interpretation deviations caused by subjective differences. Based on masking portions of medical images according to the disease process information and presenting medical images based on various color models, the medical image annotating system may obtain annotation results with high consistency and accuracy within a reasonable working time.

## Claims

1. A medical image annotation method, **characterized by** comprising:
masking a first pattern in a medical image (IMG0) to generate a first image (IMG1) according to disease process information (DI) and user indication (I1) (S210);
applying the first image (IMG1) to a plurality of color models to generate a plurality of second images (IMG2) according to the disease process information (DI), such that a user (UR) interprets symptom pattern information in the second images (IMG2) according to the disease process information (DI) to provide interpretation indication (I2) (S220); and
annotating the medical image (IMG0) according to the interpretation indication (I2) and preset annotation information to generate an annotation result (R1) (S230).

2. The medical image annotation method as claimed in claim 1, further comprising:
based on a plurality of medical images, generating a plurality of annotation results (FIGS. 8A, 8B) according to a plurality of interpretation indications corresponding to the medical images and the preset annotation information (S361, S311-S353); and
generating a plurality of guided interpretation options according to comparison results among the annotation results, such that based on the guided interpretation options (S312, S343, S353), users (UR) perform interpretation according to the disease process information(DI) (S361, S311-S353).

3. The medical image annotation method as claimed in claim 1, wherein the first pattern (411, 711) has a not-entirely-dependent relationship with a symptom structure in the disease process information (DI).

4. The medical image annotation method as claimed in claim 1, further comprising:
determining whether the medical image (IMGO, 510, 520) comprises a second pattern (511, 512) corresponding to a complete symptom structure according to the disease process information (S321).

5. The medical image annotation method as claimed in claim 4, wherein the second pattern (511, 512) is a closed region corresponding to a complete optic disc structure in a fundus image in response to the medical image (IMGO, 510, 520) being the fundus image.

6. The medical image annotation method as claimed in claim 1, further comprising:
determining whether the medical image (IMGO, 610) comprises at least one identifiable structural pattern (611, 612) according to the disease process information (DI) (S331).

7. The medical image annotation method as claimed in claim 6, wherein the at least one identifiable structural pattern (611, 612) comprises at least one of a vascular branch pattern (612) and an optic disc pattern (611) in response to the medical image (IMGO, 610) being the fundus image.

8. The medical image annotation method as claimed in claim 1, further comprising:
selecting an original color image model and a single color channel image model as the color models according to anatomical structure site information in the disease process information (DI) (S342).

9. The medical image annotation method as claimed in claim 1, further comprising:
selecting an original color image model and a contrast-enhanced image model as the color models according to symptom site information in the disease process information (DI) (S341).

10. The medical image annotation method as claimed in claim 1, wherein annotating the medical image (IMG0) according to the interpretation indication (I2) and the preset annotation information to generate the annotation result (R1) comprises:
annotating a second pattern in the medical image according to the interpretation indication, wherein the second pattern corresponds to a complete symptom structure (S353); and
determining whether the annotated medical image satisfies the preset annotation information so as to use the annotated medical image as the annotation result (R1) (S352).

11. A medical image annotating system (100), **characterized by** comprising:
a storage device (120) configured to store a medical image (IMGO); and
a processor (110) coupled to the storage device (120), and configured to execute the medical image annotation method (S210-S230) as claimed in claim 1.
